(19) Europäisches Patentamt
European Patent Office
Office européen des brevets

(11) Publication number: 0 284 405 A2

# (12) EUROPEAN PATENT APPLICATION

(21) Application number: 88302654.4

(22) Date of filing: 25.03.88

(51) Int. Cl.[4]: C 07 H 19/10
C 07 F 9/09, A 61 K 31/70

(30) Priority: 27.03.87 US 30519 20.07.87 US 75563
20.07.87 US 75902 19.11.87 US 122735
19.11.87 US 122736 25.11.87 US 126996

(43) Date of publication of application:
28.09.88 Bulletin 88/39

(84) Designated Contracting States:
AT BE CH DE ES FR GB GR IT LI LU NL SE

(71) Applicant: IVAX LABORATORIES INC.
8800 N.W. 36th Street
Miami Florida 33178 (US)

(72) Inventor: Frost, Phillip
125 East San Marino Drive
Miami Beach Florida 33139 (US)

Fishman, Jack
876 Park Avenue
New York New York 10021 (US)

(74) Representative: Ritter, Stephen David et al
Mathys & Squire 10 Fleet Street
London EC4Y 1AY (GB)

## (54) Anti-viral compounds, dosage forms and methods.

(57) Phosphate pharmaceuticals are contemplated, including dosage forms suitable for oral administration. Methods of treating acquired immune deficiency syndrome and Herpes simplex viruses through oral administration of such phosphate derivatives are further contemplated by the present invention.

EP 0 284 405 A2

**Description**

ANTI-VIRAL COMPOUNDS, DOSAGE FORMS AND METHODS

BACKGROUND OF THE INVENTION

The starting materials of the present invention, 2-amino-1,9-dihydro-[(2-hydroxy-ethoxy)methyl]-6H-purin-6-one, 3′-azidothymidine (which may also be named 3′-desoxyazidothymidine or AZT), 2′,3′-didesoxyadenosine (DDA), 2′3′-didesoxycytidine (DDC) and 2′,3′-didehydrodidesoxycytidine (2′,3′-didehydro-DDC) are known compounds having anti-viral activity. With the discovery and proliferation of contagious, serious sexually transmitted viral afflictions, anti-viral medicaments of increasing potency are actively sought.

One method of increasing potency is to increase dosage. However, the limitations of this method are quickly surpassed due to considerations limiting the feasible size of the dosage. Toxicity and swallowing ease are two of the considerations related to dosage size. Thus, anti-viral medicaments having increased potency within size and toxicity limitations are being sought.

DETAILED DESCRIPTION OF THE INVENTION

The first aspect of the present invention provides a compound of the formula

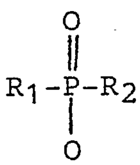

(I)

wherein $R_1$ and $R_2$ are each selected from the group consisting of the following:

(a)

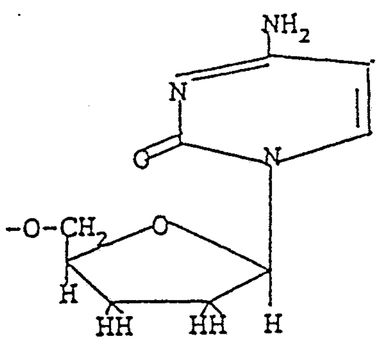

(b)

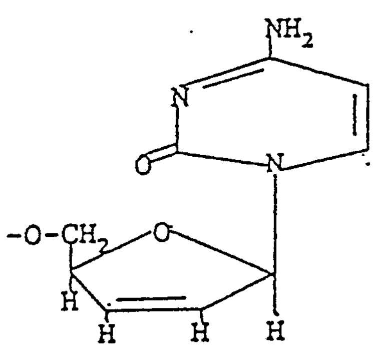

(c)

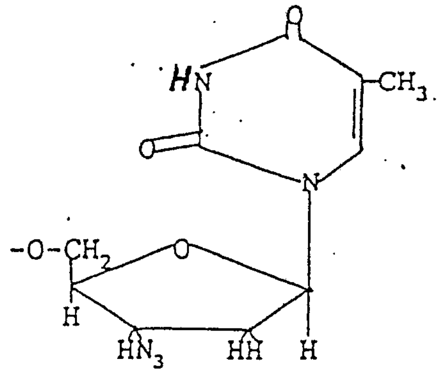

(d)

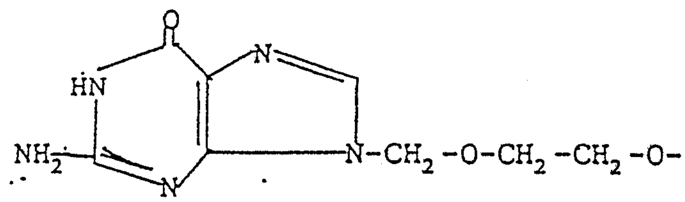

and

(e)

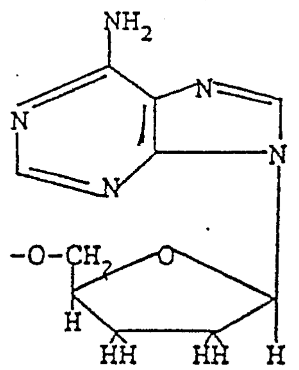

or a pharmaceutically acceptable salt of compound I. As a pharmaceutically acceptable salt there is contemplated any salt of the compound which exhibits the antivrial properties of the compound. For example, the hydrochloric acid salt of the compound of formula (I) is contemplated by the present invention.

$R_1$ and $R_2$ may be the same or different compounds selected from the above group; i.e., compound I may be a phosphate-bridged homodimer or heterodimer.

A preferred embodiment of the first aspect of the present invention provides a pharmaceutical dosage form for the oral delivery of a compound of formula (I) to a patient which includes said compound and a pharmaceutically acceptable inert ingredient, wherein said pharmaceutically acceptable inert ingredient does not interfere with the anti-viral activity of said compound.

As a dosage form for oral delivery there is contemplated any dosage form capable of being delivered orally. That is, tablets, coated tablets, capsules, caplets or any other dosage form are contemplated by the present invention.

As said pharmaceutically acceptable inert ingredients there are contemplated carriers, excipients, fillers, etc. which do not interfere with the anti-viral activity of said compound.

In addition, other pharmaceuticals such as different anti-virals or other medicaments may be included in the dosage form of the present invention. Exemplary of suitable additional anti-viral compounds are amantadine hydrochloride, idoxuridine and methisazone.

Also, fillers such as clays or siliceous earth may be utilized if desired to adjust the size of the dosage form.

Further ingredients such as excipients and carriers may be necessary to impart the desired physical properties of the dosage form. Such physical properties are, for example, release rate, texture and size. Examples of excipients and carriers useful in oral dosage forms are waxes such as beeswax, castor wax, glycowax and carnauba wax, cellulose compounds such as methylcellulose, ethylcellulose, carboxymethylcellulose, cellulose acetate phthalate, hydroxypropylcellulose and hydroxypropylmethylcellulose, polyvinyl chloride, polyvinyl pyrrolidone, stearyl alcohol, glycerin monstearate, methacrylate compounds such as polymethacrylate, methyl methacrylate and ethylene glycol dimethacrylate, polyethylene glycol and hydrophilic gums.

A more preferred embodiment of the first aspect of the present invention involves a pharmaceutical dosage form, wherein the compound of formula (I) is present in an amount between about 50 and about 800 mg. The exact dosage for each patient will be a function of the physical characteristics of that patient such as body weight.

Another preferred embodiment of the first aspect of the present invention provides a method of combatting acquired immune deficiency syndrome (AIDS) which comprises oral administration of a compound of formula (I) to a patient having AIDS, wherein said compound cleaves at the 5′ phosphate group within the body to release the anti-viral activity of each portion of said compound.

That is, the portions of the compound on either side of the phosphate bridging moiety are useful in combatting acquired immuned deficiency syndrome. However, the administration of the phosphate-bridged compound with subsequent cleavage in the body of the patient results in a level of anti-viral activity greater than that observed when each portion of the compound is administered separately.

A more preferred embodiment of this aspect involves a method, wherein said compound is administered once daily. However, the desired total daily dosage of the compound of formula (I) may be administered in divided doses.

Another aspect of the present invention involves a method of combatting Herpes simplex which comprises oral administration of a compound of formula II:

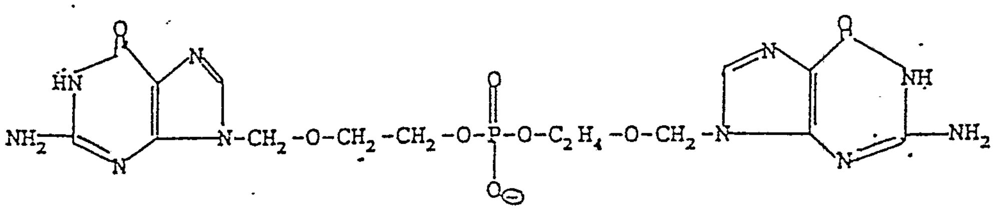

(II)

to a patient suffering from the Herpes simplex virus wherein said compound cleaves at the 5′ phosphate group within the body to release the anti-viral activity of each portion of said compound.

A more preferred embodiment of this aspect provides a method, wherein said compound is administered once daily.

The compounds of the present invention may be produced by a stepwise reaction mechanism involving phosphorylation followed by coupling of the phosphorylated group with the desired nucleic acid derivative.

The phosphorylation step is accomplished, for example, through reaction of the compound to be phosphorylated with the barium salt of 2-cyanoethylphosphate. The 2-cyanoethylphosphate compound may be prepared in accordance with the method disclosed by Tener et al., J. Amer. Soc., Vol. 83, p. 159 (1961). The reaction product thus obtained is further contacted with dicyclohexyl carbodiimide and the resultant mixture is treated through the steps of (1) addition of water, (2) evaportation in vacuo to remove pyridine, (3) filtration to remove urea, (4) addition of a 1.0 N sodium hydroxide solution, (5) heating in boiling water, (6) passing cooled through a separation column and (7) a second evaporation in vacuo.

The coupling step may be accomplished through the addition of the product of the phosphorylation step to the desired compound. The reaction product thus obtained is further contacted with dicyclohexyl carbodiimide and the resultant mixture is treated through the steps of (1) addition of water, (2) filtration to remove precipitate, (3) evaporation in vacuo and (4) isolation of the desired product through preparative thin layer chromatography.

An alternative method for the preparation of the compounds of the present invention involves the suspension of a barium salt of 2-cyanoethylphosphate in water with several grains of an acid ($H^+$) form of an ion-exchange resin, such as DOWEX 50. This mixture is stirred until the 2-cyanoethylphosphate is dissolved. Then the solution is passed through an ion-exchange column containing the same ion-exchange resin used above. The column is washed with water and the resultant liquid is treated under vacuum to give a purified oil of 2-cyanoethylphosphate.

The nucleoside starting material is admixed with the 2-cyanoethylphosphate dissolved in pyridine. Dicyclohexyl carbodiimide is then added and the resultant solution is stirred. Purification of the phosphorylated product involves the steps of evaporation of water; addition of methanol to dissolve and remove the insoluble precipitate; removal of the methanol under vacuum to yield an oil; dissolution of the oil in methanol; and purification of the solution on silica gel with the solvent being $CHCl_3$:methanol:$NH_3$ (60:40:1).

The phosphorylated product is dissolved in anhydrous pyridine and 2-mesitylenesulfonylchloride is added to the resultant solution. This mixture is stirred followed by addition of 1-methylimidazole. The resultant solution is stirred prior to the addition of the other nucleoside starting material. The product is obtained through the

purification steps of evaporation in vacuo; dissolution in $CHCl_3$:methanol (3:1); extraction with water; separation into an organic and aqueous phase; evaporation of the aqueous phase; purification of the aqueous phase through silica gel chromatography with a solvent such as $CHCl_3$:methanol:$NH_3$ (70:30:8).

Another method of preparing the compounds of the present invention involves a synthesis via a phosphoramidite intermediate. A similar process involving such an intermediate is disclosed in Beaucage, Tetrahedron Letters, vol. 25, no. 4, pp. 375-78 (1984).

A modified nucleoside starting material is admixed with 4,5-dichloroimidazole and dissolved in dry dimethylformamide. This mixture is added slowly along the wall of the flask to bis(pyrollidino)methoxyphosphine.

After 10 minutes, more of the starting nucleoside and 1H-tetrazole in methylcyanide ($CH_3CN$) are added. The mixture is then left standing for a time followed by cooling to -10°C. A solution of iodine and an inert material such as collidine or lutidine in tetrahydrofuran:water (2:1) is added to the cooled mixture. The resultant is agitated. Purification of the product involves the steps of evaporation in vacuo; dissolution of the residue in 10% n-butanol in $CHCl_3$; addition of a 5% $NaHSO_3$ solution; separation of the organic layer; wash of the aqueous layer with $CHCl_3$:methanol (3:1); evaporation in vacuo and purification by silica gel chromatography with a solvent such as $CHCl_3$:methanol:$NH_3$ (70:30:8).

The starting materials of the present invention are prepared according to known procedures. That is, 2-amino-1,9-dihydro-9-[(2-hydroxy-ethoxy)methyl]-6H-purin-6-one is prepared in accordance with German Patent 2,539,963. AZT can prepared from thymidine obtained in accordance with the teachings of Levene, J. Biol. Chem., v. 83, p. 793 (1929). DDA can be prepared from adenosine obtained in accordance with the teachings of Davoll et al., J. Chem. Soc., 1948, p. 967. DDC and 2′,3′-didehydro-DDC can be prepared from cytidine obtained in accordance with the teachings of Howard et al.,J. Chem. Soc., 1947, p. 1052.

Also in accordance with the present invention, there is provided a liquid-based dosage form suitable for the administration of the composition to a patient. Such liquid-based dosage forms are suitable for intraperitoneal, intramuscular or intravenous administration. The liquid base for this dosage form may be any liquid capable of transporting the composition into the body of a patient without disrupting the anti-viral activity of the composition or harm the patient. Exemplary of such a liquid is an isotonic solution. The isotonic solution may also contain conventional additives therein such as sugars.

The compositions of the present invention may be admixed according to known procedures using known excipients.

Representative examples of the invention follow.

EXAMPLE 1

200 mg of 3′-azidothymidine (3′-desoxyazidothymidine) which is dissolved in 10 ml dry pyridine is reacted with 1.5 ml of a 2-cyanoethylphosphate solution prepared in accordance with the method disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 3′-desoxyazidothymidine-5′-phosphate in a yield of approximately 95% based on the amount of starting materials. The oil may be further purified by preparative thin-layer chromotography. Spectral data of the product includes mass spectrum (chemical ionization-isobutane) m/e = 348 and NMR spectrum (methanol-d4): 1.98 (s); 2.4-2.6 (m); and 7.65 (s) δ.

98 mg of 3′-desoxyazidothymidine which is dissolved in 4 ml dry pyridine is added to 128 mg of the oil. 380 mg of dicyclohexyl carbodiimide is added to the reqaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desire product, starting materials and other compounds. Isolation is accomplished by preparative thin layer chromatography (chloroform:methanol:ammonia at 80:20:1). 20 mg of the product, 5′5′-(di-3′-azidothymidine)-phosphate, is obtained. Spectral data for this product includes mass spectrum (chemical ionization-isobutane) m + 1 = 597; m/e = 471, 268 and 127 and NMR spectrum (methanol-d4):1.98(s); 2.2-2.6(m); 6.1-6.5(m); and 7.65(s) δ.

EXAMPLE 2

400 mg of the compound of Example 1 is admixed with carboxymethylcellulose and sucrose. The resultant mixture is compressed into a tablet suitable for oral administration.

EXAMPLE 3

A patient suffering from acquired immune deficiency syndrome is administered a tablet of Example 2 one time daily.

EXAMPLE 4

200 mg of 2′,3′-didesoxycytidine which is dissolved in 10 ml dry pyridine is reacted with 1.5 ml of a 2-cyanoethylphosphate solution prepared in accordance with the method disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 2′,3′-didesoxycytidine-5′-phosphate in a yield of approximately 95% based on the amount of starting materials. The oil may be further purified by preparative thin-layer chromotography.

98 mg of 2′,3′-didehydrodidesoxycytidine which is dissolved in 4 ml dry pyridine is added to 128 mg of the oil. 380 mg of dicyclohexyl carbodiimide is added to the reaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desired product, starting materials and other compounds. Isolation is accomplished by preparative thin layer chromatography (chloroform:methanol:ammonia at 80:20:1). 20 mg of the product 5′-(2′,3′-didesoxycytidine)-5′-(2′,3′-didehydrodidesoxycytidine)phosphate, is obtained.

EXAMPLE 5

600 mg of the compound of Example 4 is admixed with polyethylene glycol and hydroxypropylcellulose. The resultant mixture is compressed into a tablet suitable for oral administration.

EXAMPLE 6

A patient suffering from acquired immune deficiency syndrome is administered a tablet of Example 5 one time daily.

EXAMPLE 7

200 mg of 2′,3′-didehydrodidesoxycytidine which is dissolved in 10 ml dry pyridine is reacted with 1.5 ml of a 2-cyanoethylphosphate solution prepared in accordance with the method disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 2′,3′-didehydrodidesoxycytidine-5′-phosphate in a yield of approximately 95% based on the amount of starting materials. The oil may be further purified by preparative thin-layer chromotography.

98 mg of 2-amino-1,9-dihydro-9-[(2-hydroxy-ethoxy)methyl]-6H-purin-6-one which is dissolved in 4 ml dry pyridine is added to 128 mg of the oil. 380 mg of dicyclohexyl carbodiimide is added to the reaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desired product, starting materials and other compounds. Isolation is accomplished by preparative thin layer chromatography (chloroform:methanol:ammonia at 80:20:1). 20 mg of the product, 5′-(2′,3′-didehydrodidesoxycytidine)-5′-{2-amino-1,9-dihydro-9-[(2-hydroxy-ethoxy)methyl]-6H-purine-6-one}phosphate, is obtained.

EXAMPLE 8

800 mg of the compound of Example 7 is admixed with methyl cellulose and glycerin monostearate. The resultant mixture is compressed into a tablet suitable for oral administration.

EXAMPLE 9

A patient suffering from acquired immune deficiency syndrome is administered a tablet of Example 8 one time daily.

EXAMPLE 10

200 mg of 2-amino-1,9-dihydro-9-[(2-hydroxy-ethoxy)methyl]-6H-purin-6-one which is dissolved in 10 ml dry pyridine is reacted with 1.5 ml of a 2-cyanoethylphosphate solution prepared in accordance with the method

disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 2-amino-1,9-dihydro-9-[(2-hydroxy-ethoxy)methyl]-6H-purin-6-one-5′-phosphate in a yield of approximately 95% based on the amount of starting materials. The oil may be further purified by preparative thin-layer chromotography.

98 mg of 3′-azidothymidine(3′-desoxyazidothymidine) which is dissolved in 4 ml dry pyridine is added to 128 mg of the oil. 380 mg of dicyclohexyl carbodiimide is added to the reaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desired product, starting materials and other compounds. Isolation is accomplished by preparative thin layer chromatography (chloroform:methanol:ammonia at 80:20:1). 20 mg of the product, 5′-{2-amino-1,9-dihydro-9-[(2-hydroxy-ethoxy)methyl]-6H-purin-6-one}-5′-(3-azidothymidine)phosphate, is obtained. Spectral data of this product includes mass spectrum(fission fragment ) $(M)^+ = 554.134$; $(M+Na)^+ = 577.129$ and NMR spectrum (methanol-d4 and $D_2O$): 1.95 (s); 2.2-2.6 (m); 4.1-4.5 (m); 6.4-6.6 (m); and 7.8 (s) δ.

EXAMPLE 11

200 mg of the compound of Example 10 is admixed with hydroxypropylcellulose and sucrose. The resultant mixture is compressed into a tablet suitable for oral administration..

EXAMPLE 12

A patient suffering from acquired immune deficiency syndrome is administered a tablet of Example 11 one time daily.

EXAMPLE 13

200 mg of 2-amino-1,9-dihydro-9-[(2-hydroxy-ethoxy)methyl]-6H-purin-6-one which is dissolved in 10 ml dry pyridine is reacted with 1.5 ml of a 2-cyanoethylphosphate solution prepared in accordance with the method disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 2-amino-1,9-dihydro-9-[(2-hydroxy-ethoxy)methyl]-6H-purin-6-one-5′-phosphate in a yield of approximately 95% based on the amount of starting materials. The oil may be further purified by preparative thin-layer chromotography.

98 mg of 2-amino-1,9-dihydro-9-[(2-hydroxy-ethoxy)methyl]-6H-purin-6-one which is dissolved in 4 ml dry pyridine is added to 128 mg of the oil. 380 mg of dicyclohexyl carbodiimide is added to the reaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desired product, starting materials and other compounds. Isolation is accomplished by preparative thin layer chromatography (chloroform:methanol:ammonia at 80:20:1). 20 mg of the product, 5′,5′-di-{2-amino-1,9-dihydro-9-[(2-hydroxy-ethoxy)methyl]-6H-purin-6-one}phosphate, is obtained.

EXAMPLE 14

400 mg of the compound of Example 13 is admixed with carboxymethylcellulose and sucrose. The resultant mixture is compressed into a tablet suitable for oral administration.

EXAMPLE 15

A patient suffering from Herpes simplex virus is administered a tablet of Example 14 one time daily.

EXAMPLE 16

200 mg of 2′,3′-didesoxyadenosine which is dissolved in 10 ml dry pyridine is reacted with 1.5 ml of a 2-cyanoethylphosphate solution prepared in accordance with the method disclosed by Tener et al., J. Amer. Chem. Soc., Vol. 83, p. 159 (1961). 3 mmol dicyclohexyl carbodiimide is then added to the reaction vessel. The reaction product thus obtained is then stirred at room temperature for 18 hours.

Then 3 ml of water is added and the resultant mixture is stirred at room temperature for 30 minutes. Evaporation in vacuo to remove pyridine is then undertaken. 15 ml of water is added to the evaporated product and filtration is undertaken to remove the by-product urea from the product. The precipitate is washed with 10 ml water. Next, 15 ml of a 1.0 N solution of sodium hydroxide is added. Heating in boiling water for 40 minutes is then commenced. The product is then cooled to room temperature and passed through a Dowex 50 $H^+$ column (Dow Chemical Company), washed in 45 ml of water and evaporated in vacuo to yield 325 mg of an oil product. This oil product contains 2′,3′-didesoxyadenosine-5′-phosphate in a yield of approximately 95% based on the amount of starting materials. The oil may be further purified by preparative thin-layer chromotography.

98 mg of 2′,3′-didesoxycytidine which is dissolved in 4 ml dry pyridine is added to 128 mg of the oil. 380 mg of dicyclohexyl carbodiimide is added to the reaction vessel. The reaction product thus obtained is stored at room temperature for 20 hours.

Then 10 ml of water is added to the admixture, and 30 minutes of stirring is commenced. Filtration is then undertaken to remove a precipitate, and evaporation of the product in vacuo yields 260 mg of an oil containing a mixture of the desired product, starting materials and other compounds. Isolation is accomplished by preparative thin layer chromatography (chloroform:methanol:ammonia at 80:20:1). 20 mg of the product, 5′-(2′,3′-didesoxyadenosine)-5′-(2′,3′-didesoxycytidine)phosphate, is obtained.

EXAMPLE 17

600 mg of the compound of Example 16 is admixed with carboxymethylcellulose and sucrose. The resultant mixture is compressed into a tablet suitable for oral administration.

EXAMPLE 18

A patient suffering from acquired immune deficiency syndrome is administered a tablet of Example 17 one time daily.

EXAMPLE 19

3 grams of the barium salt of 2-cyanoethylphosphate is suspended in water with several grains of DOWEX 50. The solution is agitated until the 2-cyanoethylphosphate is dissolved. The solution is then passed through a DOWEX 50 column. The column is washed with 60 ml of water and the resultant liquid is treated under vacuum to yield 941 mg of a purified oil of 2-cyanoethylphosphate.

1.8 mmoles 3′-azidothymidine (3′-desoxyazidothymidine) dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to product 0.9 grams of an oil. The oil is then dissolved in 10 ml of methanol and purified further on silica gel with 60:40:1 of $CHCl_3$:methanol:$NH_3$ to give 1.4 mmoles of a phosphorylated product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2′,3′-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5′-(3′-azidothymidine)-5′-(2′,3′-didesoxyadenosine)phosphate. Spectral data of this product includes mass spectrum (fission fragment ) $(M+H)^+ = 565.168$; $(M+Na)^+ = 587.150$; $(M+2Na)^+ = 609.132$; and $(M+3Na)^+ = 631.114$ and NMR spectrum: 1.98 (s); 2.2-2.5 (m); 4.1-4.6 (m); 6.1-6.3 (m); 7.7 (s); 8.2 (s); and 8.45 (s) $\delta$ .

EXAMPLE 20

400 mg of the compound of Example 19 is admixed with carboxymethylcellulose and sucrose. The resultant mixture is compressed into a tablet suitable for oral administration.

EXAMPLE 21

A patient suffering from acquired immune deficiency syndrome is administered a tablet of Example 20 one time daily.

EXAMPLE 22

3 grams of the barium salt of 2-cyanoethylphosphate is suspended in water with several grains of DOWEX 50. The solution is agitated until the 2-cyanoethylphosphate is dissolved. The solution is then passed through a

DOWEX 50 column. The column is washed with 60 ml of water and the resultant liquid is treated under vacuum to yield 941 mg of a purified oil of 2-cyanoethylphosphate.

1.8 mmoles 2′,3′-didesoxycytidine dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to produce 0.9 grams of an oil. The oil is then dissolved in 10 ml of methanol and purified further on silica gel with 60:40:1 of $CHCl_3$:methanol:$NH_3$ to give 1.4 mmoles of a phosphorylated 2′,3′-didesoxycytidine product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2′,3′-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5′-(2′,3′-didesoxycytidine)-5′-(2′,3′-didesoxyadenosine)phosphate.

EXAMPLE 23

400 mg of the compound of Example 22 is admixed with carboxymethylcellulose and sucrose. The resultant mixture is compressed into a tablet suitable for oral administration.

EXAMPLE 24

A patient suffering from acquired immune deficiency syndrome is administered a tablet of Example 23 one time daily.

EXAMPLE 25

3 grams of the barium salt of 2-cyanoethylphosphate is suspended in water with several grains of DOWEX 50. The solution is agitated until the 2-cyanoethylphosphate is dissolved. The solution is then passed through a DOWEX 50 column. The column is washed with 60 ml of water and the resultant liquid is treated under vacuum to yield 941 mg of a purified oil of 2-cyanoethylphosphate.

1.8 mmoles 2′,3′-didehydrodidesoxycytidine dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to product 0.9 grams of an oil. The oil is then dissolved in 10 ml of methanol and purified further on silica gel with 60:40:1 of $CHCl_3$:methanol:$NH_3$ to give 1.4 mmoles of a phosphorylated 2′,3′-didehydrodidesoxycytidine product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2′,3′-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5′-(2′,3′-didehydrodidesoxycytidine)-5′-(2′,3′-didesoxyadenosine)phosphate.

EXAMPLE 26

400 mg of the compound of Example 25 is admixed with carboxymethylcellulose and sucrose. The resultant mixture is compressed into a tablet suitable for oral administration.

EXAMPLE 27

A patient suffering from acquired immune deficiency syndrome is administered a tablet of Example 26 one time daily.

EXAMPLE 28

3 grams of the barium salt of 2-cyanoethylphosphate is suspended in water with several grains of DOWEX 50. The solution is agitated until the 2-cyanoethylphosphate is dissolved. The solution is then passed through a DOWEX 50 column. The column is washed with 60 ml of water and the resultant liquid is treated under vacuum to yield 941 mg of a purified oil of 2-cyanoethylphosphate.

1.8 mmoles 2-amino-1,9-dihydro-9-[(2-hydroxy-ethoxy)methyl]-6H-purin-6-one dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the

solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to produce 0.9 grams of an oil. The oil is then dissolved in 10 ml of methanol and purified further on silica gel with 60:40:1 of $CHCl_3$:methanol:$NH_3$ to give 1.4 mmoles of a phosphorylated 2-amino-1,9-dihydro-9-[(2-hydroxy-ethoxy)methyl]-6H-purin-6-one product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2',3'-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5'-{2-amino-1,9-dihydro-9-[(2-hydroxy-ethoxy)methyl]-6H-purin-6-one}-5'-(2',3'-didesoxyadenosine)phosphate.

EXAMPLE 29

400 mg of the compound of Example 28 is admixed with carboxymethylcellulose and sucrose. The resultant mixture is compressed into a tablet suitable for oral administration.

EXAMPLE 30

A patient suffering from acquired immune deficiency syndrome is administered a tablet of Example 29 one time daily.

EXAMPLE 31

3 grams of the barium salt of 2-cyanoethylphosphate is suspended in water with several grains of DOWEX 50. The solution is agitated until the 2-cyanoethylphosphate is dissolved. The solution is then passed through a DOWEX 50 column. The column is washed with 60 ml of water and the resultant liquid is treated under vacuum to yield 941 mg of a purified oil of 2-cyanoethylphosphate.

1.8 mmoles 2-amino-1,9-dihydro-9-[(2-hydroxy-ethoxy)methyl]-6H-purin-6-one dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to produce 0.9 grams of an oil. The oil is then dissolved in 10 ml of methanol and purified further on silica gel with 60:40:1 of $CHCl_3$:methanol:$NH_3$ to give 1.4 mmoles of a phosphorylated 2-amino-1,9-dihydro-9-[(2-hydroxy-ethoxy)methyl]-6H-purin-6-one product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2',3'-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5'-{2-amino-1,9-dihydro-9-[(2-hydroxy-ethoxy)methyl]-6H-purin-6-one}-5'-(2',3'-didesoxyadenosine)phosphate.

EXAMPLE 32

400 mg of the compound of Example 31 is admixed with carboxymethylcellulose and sucrose. The resultant mixture is compressed into a tablet suitable for oral administration.

EXAMPLE 33

A patient suffering from acquired immune deficiency syndrome is administered a tablet of Example 32 one time daily.

EXAMPLE 34

3 grams of the barium salt of 2-cyanoethylphosphate is suspended in water with several grains of DOWEX 50. The solution is agitated until the 2-cyanoethylphosphate is dissolved. The solution is then passed through a DOWEX 50 column. The column is washed with 60 ml of water and the resultant liquid is treated under vacuum to yield 941 mg of a purified oil of 2-cyanoethylphosphate.

1.8 mmoles 2',3'-didesoxyadenosine dissolved in 40 ml of pyridine is added to the 941 mg of 2-cyanoethylphosphate. It is not necessary that all of the 2-cyanoethylphosphate dissolve in the solution. 1.482 grams of dicyclohexyl carbodiimide is added to the solution, and the resultant is stirred at room temperature for 18 hours. The water in the solution is evaporated to obtain a gum. Methanol is added to dissolve the gum and 120 mg of an insoluble precipitate is removed. The methanol is evaporated in vacuo to produce 0.9 grams of an oil. The oil is then dissolved in 10 ml of methanol and purified further on silica gel with 60:40:1 of

$CHCl_3$:methanol:$NH_3$ to give 1.4 mmoles of a phosphorylated 2',3'-didesoxyadenosine product.

0.13 mmoles of this product are dissolved in 1 ml of anhydrous pyridine and 0.27 mmoles of 2-mesitylenesulfonylchloride are added. The mixture is stirred for 30 minutes at room temperature. 0.54 mmoles of 1-methylimidazole are then added and the resultant is agitated at room temperature for 1 hour. 0.14 mmoles of 2',3'-didesoxyadenosine is then added to the agitated solution. The mixture is agitated at room temperature for 18 hours followed by evaporation in vacuo. The evaporated product is dissolved in a solution 3:1 of $CHCl_3$:methanol and extracted with water to separate the mixture into an organic phase and an aqueous phase. The aqueous phase is then evaporated and purified using silica gel with a 70:30:8 solvent of $CHCl_3$:methanol:$NH_3$ to give 0.04 mmoles of 5',5'-di-(2',3'-didesoxyadenosine)phosphate.

Spectral data for this product includes mass spectrum (fission fragment ) $(M)^+ = 532.170$; $(M+Na)^+ = 555.160$; $(M+2Na\text{-}H)^+ = 577.142$; $(M+3Na\text{-}2H)^+ = 599.124$ and NMR spectrum (methanol-d4): 2-2.5 (m); 3.4 (s) 4.15 (s) and 6.38 (s) δ.

EXAMPLE 35

400 mg of the compound of Example 34 is admixed with carboxymethylcellulose and sucrose. The resultant mixture is compressed into a tablet suitable for oral administration.

EXAMPLE 36

A patient suffering from acquired immune deficiency syndrome is administered a tablet of Example 35 one time daily.

EXAMPLE 37

0.28 mmole 3'-azidothymidine (3'-desoxyazidothymidine) is added to 1 mmole of 4,5-dichloroimidazole and dissolved in 0.5 ml of dry dimethylformamide. This mixture is added slowly to 50 microliters of bis-(pyrollidino)methoxyphosphine via addition along the wall of the flask. After 10 minutes pass, another 0.28 mmoles of 3'-azidothymidine (3'-desoxyazidothymidine) and 0.9 mmoles of 1H-tetrazole dissolved in 2.5 ml $CH_3CN$ are added. The mixture is left standing at room temperature for 10 minutes then is cooled to -10°C. A solution containing 0.28 mmoles of iodine and 0.498 mmoles of collidine in 2:1 tetrahydrofuran:water solution is added. The mixture is then agitated for 10 minutes prior to evaporation in vacuo. The residue is dissolved in 2 ml of a 10% n-butanol in $CHCl_3$ solution. 5% $NaHSO_3$ solution is added until the iodine color is discharged. The organic layer is then separated and the aqueous layer is washed with $CHCl_3$ and $CHCl_3$:methanol (3:1) successively. Evaporation in vacuo to give 450 mg of oil followed by purification by silica gel chromatography with a solvent of $CHCl_3$:methanol:$NH_3$ of 70:30:8 to yield 5'5'-(di-3'-azidothymidine)-phosphate.

Spectral data for this product is identical to that of Example 1.

EXAMPLE 38

400 mg of the compound of Example 37 is admixed with carboxymethylcellulose and sucrose. The resultant mixture is compressed into a tablet suitable for oral administration.

EXAMPLE 39

A patient suffering from acquired immune deficiency syndrome is administered a tablet of Example 38 one time daily.

EXAMPLE 40

The procedure of Example 37 is repeated except that 2',3'-didesoxyadenosine is used to yield 5'-(3'-azidothymidine)-5'-(2',3'-didesoxyadenosine)phosphate.

Spectral data for this product is identical to that of Example 19.

EXAMPLE 41

400 mg of the compound of Example 40 is admixed with carboxymethylcellulose and sucrose. The resultant mixture is compressed into a tablet suitable for oral administration.

EXAMPLE 42

A patient suffering from acquired immune deficiency syndrome is administered a tablet of Example 41 one time daily.

EXAMPLE 43

The procedure of Example 37 is repeated except that 2',3'-didesoxycytidine is used to yield 5'-(3'-azidothymidine)-5'-(2',3'-didesoxycytidine)phosphate.

EXAMPLE 44

400 mg of the compound of Example 43 is admixed with carboxymethylcellulose and sucrose. The resultant mixture is compressed into a tablet suitable for oral administration.

EXAMPLE 45

A patient suffering from acquired immune deficiency syndrome is administered a tablet of Example 44 one time daily.

EXAMPLE 46

The procedure of Example 37 is repeated except that 2′,3′-didehydrodidesoxycytidine is used to yield 5′-(3′-azidothymidine)-5′-(2′,3′-didehydrodidesoxycytidine)phosphate.

EXAMPLE 47

400 mg of the compound of Example 46 is admixed with carboxymethylcellulose and sucrose. The resultant mixture is compressed into a tablet suitable for oral administration.

EXAMPLE 48

A patient suffering from acquired immune deficiency syndrome is administered a tablet of Example 47 one time daily.

EXAMPLE 49

The procedure of Example 37 is repeated except that 2-amino-1,9-dihydro-9-[(2-hydroxy-ethoxy)methyl]-6H-purin-6-one is used to yield 5′-(3′-azidothymidine)-5′-{2-amino-1,9-dihydro-9-[{2-hydroxy-ethoxy)methyl]-6H-purin-6-one)phosphate. Spectral data for this product is identical to that of Example 10.

EXAMPLE 50

400 mg of the compound of Example 49 is admixed with carboxymethylcellulose and sucrose. The resultant mixture is compressed into a tablet suitable for oral administration.

EXAMPLE 51

A patient suffering from acquired immune deficiency syndrome is administered a tablet of Example 47 one time daily.

EXAMPLE 52

1. Preparation of Target Mononuclear Cells

Normal peripheral blood (NPB) mononuclear cells are obtained by leukophoresis of healthy adults, are banded by ficol-paque gradient centrifugation and are grown in growth medium (RPMI 1640; 20% heat inactivated fetal calf serum and 0.25 mg of glutamine/ml) containing 5 microgram/ml phytohemagglutinin for 48 hours at 37°C in a 5% $CO_2$ atmosphere. These cells are then grown in growth medium containing 10% interleukin-2.

2. Preparation of HIV (TM) Stock

An isolate of HIV (TM) is obtained from the culture supernatant of NPB mononuclear cells infected with the virus isolated from a patient with AIDS. These HIV (TM) stocks have an infectious titer of approximately 7 log 10 TCID 50/ml, are filtered and are kept frozen at -85°C until use.

3. HIV Infectivity Assay

Target mononuclear cells are seeded at a concentration of $10^6$ cells/ml and are exposed to the drug to be tested for 24 hours. The drugs to be tested are:

(1) azidothymidine;

(2) 2′,3′-dideoxyadenosine;

(3) a compound of formula (I), wherein $R_1$ and $R_2$ are both (e);

(4) a compound of formula (I ), wherein $R_1$ and $R_2$ are both (c);

(5) 50:50 weight ratio combination of (1) and (2); and

(6) a compound of formula (I), wherein $R_1$is (c) and $R_2$ is (e).These exposed cells are then infected with an amount of the HIV stock corresponding to a 0.1 and 1.0 multiplicity of infection units (MOI, number of infectious viral particles/cell).

After this incubation step, NPB cells are suspended in fresh culture medium supplemented with interleukin-2. H9 cells are suspended in growth medium and all other cells are carried at 37°C in humidified air containing $CO_2$. All cells are continuously exposed to 1 microgram/ml of the drug during each media change which occurs every 3-4 days. Also at each media change, viable cells are measured by the trypan-blue exclusion method and the cell density is adjusted to $10^6$/ml.

HIV-induced formation of syncytia which is recognized as 5 nuclei/cell is quantified by microscopic observation before and after staining with Wright-Geimsa. Reverse transcriptase activity and HIV p24 in cell-free supernatant fluids are assayed. Also, immunofluorescence studies utilizing an HIV p24 monoclonal antibody to determine infected cells are also performed. The results are summarized in the Table.

TABLE

| Drug* | MOI | Evaluation Period (Days)* | | | |
|---|---|---|---|---|---|
| | | 7 | 10 | 14 | 20 |
| Control | 0.1 | - | + | + | + |
| (No drug) | 1 | + | + | + | o |
| (1) | 0.1 | - | - | - | - |
| 1 g/ml | 1 | - | - | +$^{o}$ | + |
| (2) | 0.1 | - | - | - | - |
| 1 g/ml | 1 | - | - | - | - |
| (1) | 0.1 | - | - | + | + |
| 0.1 g/ml | 1 | - | + | + | o |
| (2) | 0.1 | - | - | + | + |
| 0.1 g/ml | 1 | - | + | + | o |
| (3) | 0.1 | - | - | - | - |
| 1 g/ml | 1 | - | - | + | + |
| (4) | 0.1 | - | - | - | - |
| 1 g/ml | 1 | - | - | + | + |

TABLE CONTINUED

| Drug* | MOI | Evaluation Period (Days) | | | |
|---|---|---|---|---|---|
| | | 7 | 10 | 14 | 20 |
| (5) | 0.1 | - | - | - | - |
| 1 g/ml | 1 | - | - | - | - |
| (5) | 0.1 | - | - | - | - |
| 0.2 g/ml | 1 | - | - | - | + |
| (5) | 0.1 | - | - | - | - |
| 0.1 g/ml | 1 | - | - | + | + |
| (6) | 0.1 | - | - | - | - |
| 0.1 g/ml | 1 | - | - | - | - |
| (6) | 0.1 | - | - | - | - |
| 0.02 g/ml | 1 | - | +° | + | + |

*Experiments are performed in 4 replicate cultures and the results are expressed in mean values.
The characters are defined as follows:

(+) evidence of virus production, i.e., elevation in reverse transcriptase activity, detection of HIV p24 antigen and cell cytopathic effects.

(-) no evidence of virus production.

(+°) two of the four replicate cultures were positive for virus production.

**Claims**

1. A compound of the formula:

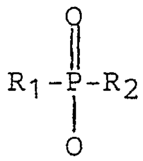

(I)

wherein $R_1$ and $R_2$ are each a member selected from the group consisting of the following:

(a)

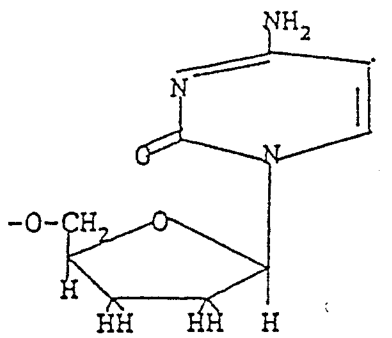

(b)

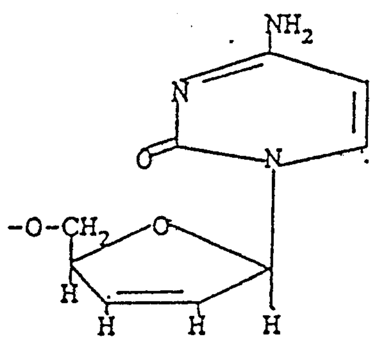

(c)

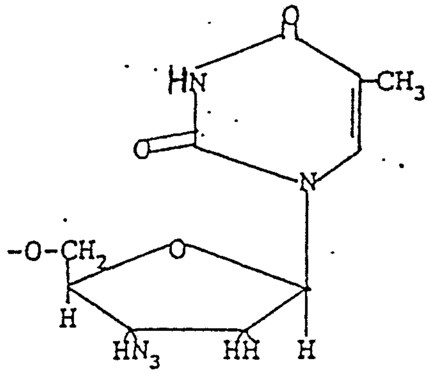

(d)

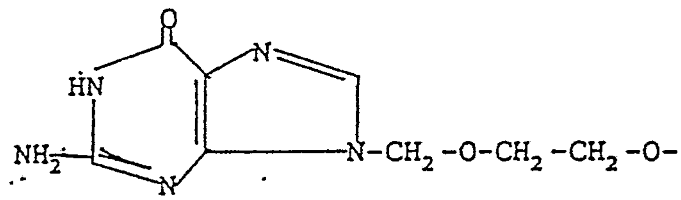

and

(e)

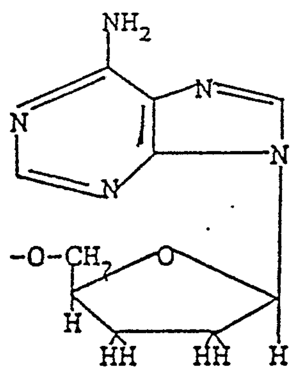

or a pharmaceutically acceptable salt thereof.

2. A pharmaceutical dosage form for the oral delivery of a compound of claim 1 to a patient which includes said compound and a pharmaceutically acceptable inert ingredient, wherein said pharmaceutically acceptable inert ingredient does not interfere with the anti-viral activity of said compound.

3. A pharmaceutical dosage form of claim 2, wherein said compound is present in an amount between about 50 and about 800 mg.

4. A method of combatting acquired immune deficiency syndrom which comprises oral administration of a compound of claim 1 to a patient having acquired immune deficiency syndrome, wherein said compound cleaves at the 5′ phosphate group within the body to release the anti-viral activity of each portion of said compound.

5. A method of claim 4, wherein said compound is administered once daily.

6. A method of combatting Herpes simplex which comprises oral administration of a compound of formula:

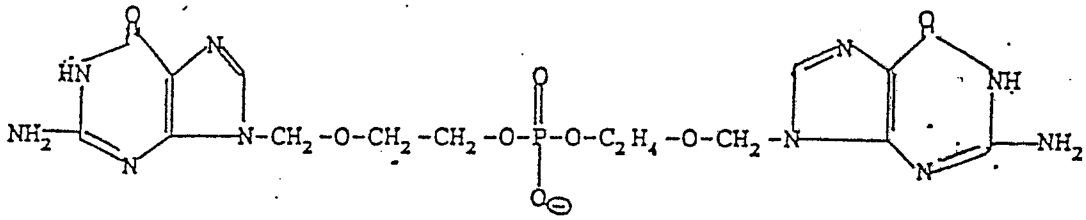

to a patient suffering from the Herpes simplex virus wherein said compound cleaves at the 5′ phosphate group within the body to release the anti-viral activity of each portion of said compound.

7. A method of claim 6, wherein said compound is administered once daily.

8. The use of a compound of general formula (I) as defined in Claim 1 in the manufacture of a medicament for use in the methods defined in any of Claims 4 to 7.